# EUROPEAN PATENT APPLICATION

(11) **EP 4 245 261 A1**
(43) Date of publication of application: **20.09.2023**
(21) Application number: 21892091.6
(22) Date of filing: 27.08.2021
(51) Int. Cl.: A61F 2/30, A61F 2/38

(54) **MODULAR AUGMENT**

(30) Priority: 10.11.2020 KR 20200149229
(71) Applicant: Corentec Co., Ltd., Seoul 06649 (KR)
(72) Inventor: IN, Yong, Seoul 06215 (KR); KIM, Joing-Min, Seoul 06215 (KR); SIM, Jae-Ang, Incheon 21561 (KR); PARK, Jun-Kyu, Cheonan-si Chungcheongnam-do 31055 (KR); JANG, Young-Woong, Seoul 04939 (KR); HAN, Ah-Reum, Hanam-si Gyeonggi-do 12923 (KR); KIM, Jung-Sung, Seoul 05555 (KR)
(74) Representative: Witte, Weller & Partner Patentanwälte mbB
(86) International application number: PCT/KR2021/011513
(87) International publication number: WO 2022/102921

(57) **Abstract**

Proposed is a modular augment, and, more specifically, a modular augment in which an additional unit is separably coupled to a center unit so as to selectively couple the additional unit to the center unit according to the degree of a patient's bone loss so that the degree of bone reinforcement can be easily adjusted, and thus a manufacturer is capable of covering various types of patients even though the types of units are minimized, thereby reducing manufacturing costs, the number of products to be prepared for surgery by a user such as a hospital is remarkably reduced, thereby preventing the unnecessary costs and facilitating inventory management, and a patient can receive an optimal augment that is appropriate for the patient' condition.

## Description

### Technical Field

The present disclosure relates to a modular augment and, more specifically, to a modular augment in which an additional unit is separably coupled to a center unit so as to selectively couple the additional unit to the center unit according to the degree of a patient's bone loss so that the degree of bone reinforcement can be easily adjusted, and thus a manufacturer is capable of covering various types of patients even though the types of units are minimized, thereby reducing manufacturing costs, the number of products to be prepared for surgery by a user such as a hospital is remarkably reduced, thereby preventing unnecessary costs and facilitating inventory management, and a patient can receive an optimal augment that is appropriate for the patient's condition.

### Background Art

A joint is a part of the body in which two or more bones are in contact with each other in a movable structure. Human joints may have various problems due to damage from repetitive use, arthritis, and aging, etc. When a joint is damaged enough to lose an original function thereof, artificial joint replacement surgery may be performed to replace the damaged joint with an implant.

Artificial joint replacement consists of manufacturing an implant having a shape complementary to the anatomical shape of a damaged joint, cutting the bone of the damaged joint within a predetermined range, and implanting the implant to a cut portion.

In this case, when a bone cutting portion is required to be removed extensively or locally, such as when the portion of the damaged joint is extensive, when there is severe local damage, or when revision surgery is required, an augment may be used to compensate for the bone portion lost by cutting.

The augment is a component to supplement a lost bone, and is configured to have a shape complementary the shape of the lost bone, so the augment is seated in an empty space defined by a resected bone.

In general, the augment may be formed in a solid form by using biomaterials (biometal, bioceramic, and biopolymer), etc.

FIG. 1 is a view illustrating a conventional tibial augment 90, which is disclosed in US Patent No. 8,506,645B2 (2013.08.13.) .

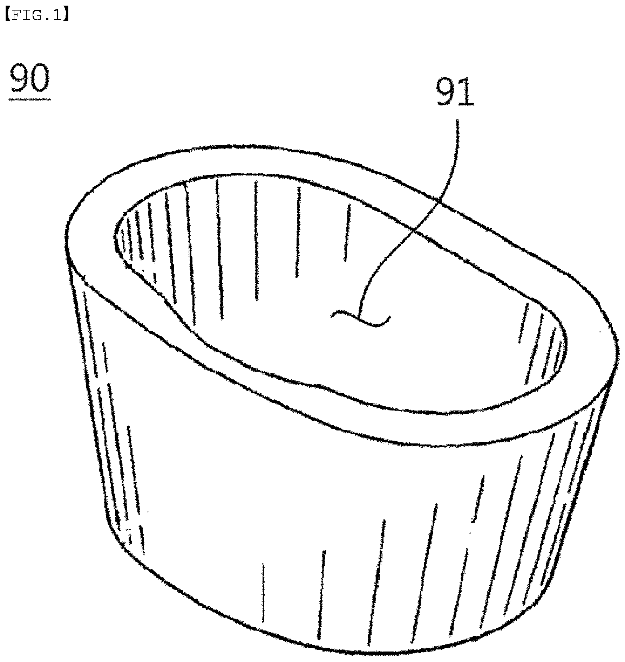

Referring to FIG. 1, the conventional tibial augment 90, which is formed in the shape of a ring made of porous metal material, has a passage formed in an inner wall thereof to accommodate a stem of a tibial implant, and an outer wall thereof is formed to be distally tapered in medial and lateral surfaces.

However, the conventional tibial augment 90, which is composed of a single body, has a shape in which the augment 90 is coupled to the tibial implant, and thus has a limitation in that the augment 90 cannot be transformed to be used according to a patient's situation.

Despite difference in the shape and scope of damaged bones depending on patients, the conventional tibial augment 90 did not reflect this, so a batch of augment was inevitably applied, resulting in bone cutting more than necessary, so there was a problem in that a large amount of bone in a normal state was removed.

In particular, in the case of revision surgery in which the conventional implant is removed and replaced with a new implant, a large amount of bone loss occurs during the process of removing the conventional implant, and further, as additional bone loss occurred due to the application of the batch of augment, the above-mentioned problem became more prominent.

This problem may occur not only with the tibial augment, but also with a femoral augment. In the related industry, there is a demand for the introduction of new technologies that can minimize bone loss and enables optimal replacement surgery by allowing the augment to be transformed and used according to a patient's situation.

(Patent Document 1) US Patent No. 8,506,645B2 (2013.08.13.)

### Disclosure

### Technical Problem

The present disclosure has been made to solve the above problems, and
an objective of the present disclosure is to provide a modular type augment which has an additional unit separably coupled to a center unit to selectively couple the additional unit to the center unit according to the degree of a patient's bone loss so that the degree of bone reinforcement can be easily adjusted.

Another objective of the present disclosure is that when a patient's bone loss is large only on a medial side, a modular augment having an additional unit coupled to the medial side of the center unit is provided, when a patient's bone loss is concentrated on a lateral side, a modular augment having an additional unit coupled to the lateral side of the center unit is provided, and when a patient's bone loss is large on both the medial and lateral sides, a modular augment having additional units coupled to the medial and lateral sides of the center unit is provided.

Still another objective of the present disclosure is that additional units coupled to the medial and lateral sides of the center unit can be configured to have different sizes so that a customized augment can be provided to a patient having different degrees of bone losses on the medial and lateral sides.

Still another objective of the present disclosure is that a surgeon can transform an augment according to a patient's condition so as to enable easy revision surgery.

Still another objective of the present disclosure is that a manufacturer can provide an augment which can cover various types of patients even if the types of units are minimized so that manufacturing costs can be reduced, the number of products to be prepared for surgery by a user such as a hospital is remarkably reduced to prevent the unnecessary costs and facilitate inventory management, and a patient can receive can an optimal augment that is appropriate for the patient's condition.

Still another objective of the present disclosure is that the additional unit is slid in a first direction so that the additional unit is coupled to the center unit, and the separation of the additional unit coupled to the center unit is performed only by sliding the additional unit in a second direction opposite to the first direction, so that although the additional unit is separably coupled to the center unit, due to the implant located in the second direction of an augment, the unintentional separation of the additional unit from an implanted augment is prevented.

Still another objective of the present disclosure is that the center unit has a cut part formed on a side surface thereof so that the additional unit can be separably inserted into the center unit through the cut part.

Still another objective of the present disclosure is that a locking part is formed on a coupling directional end part of the additional unit, so that the additional unit inserted through the cut part moves in a coupling direction and is finally fastened and fixed to the locking part.

Still another objective of the present disclosure is that the locking part includes a first vertical part protruding in the separating direction of the additional unit so that the locking part is accommodated in the additional unit, a first recessed part recessed in the coupling direction of the additional unit to accommodate the additional unit therein, and a second vertical part protruding in the separating direction of the additional unit to support the additional unit accommodated in the first recessed part so that the center unit and the additional unit are separably dovetail-coupled to each other.

Still another objective of the present disclosure is that a coupling part is formed on each of the medial and lateral sides of the center unit so that the coupling part is offset to a posterior side by a predetermined angle so that the augment having the additional unit coupled to the center unit has a shape corresponding to the anatomical shape of a cancellous bone.

Still another objective of the present disclosure is that the coupling part is formed in a non-porous structure so that the coupling strength of a coupling portion is increased and the overall rigidity of the augment is reinforced.

Still another objective of the present disclosure is that the center unit is divided into a bone contact part in contact with a bone and a bone non-contact part which is not in contact with the bone so that the bone contact part is formed in a porous structure and the bone non-contact part is formed in a non-porous structure, and bone cement is inserted into the augment so as to induce strong coupling between the augment and the implant and to fuse the implanted augment and the bone to each other due to the promotion of spontaneous bone growth therebetween.

Still another objective of the present disclosure is that the additional unit has an inclined part tapered in the coupling direction of the additional unit so that an implanted augment is located in a cortical bone.

Still another objective of the present disclosure is that a first inclined part of the additional unit tapered at a first angle relative to the vertical axis of the additional unit, and a second inclined part tapered by extending from the end of the first inclined part at a second angle relative to the vertical axis of the additional unit are provided, and the first angle is smaller than the second angle, so that the anatomical shape of a bone having a part whose cross section rapidly decreases when the part moves away from a joint surface is reflected in the augment.

Still another objective of the present disclosure is that the inclined part of the additional unit is formed to have a porous structure so as to realize the coupling of a bone and the augment through the promotion of natural bone growth in a portion of the additional unit in contact with the bone.

Still another objective of the present disclosure is that the additional unit has a fastening part so that the fastening part is separably coupled to the coupling part of the center unit.

Still another objective of the present disclosure is that the fastening part has a holding surface and when the additional unit is coupled to the center unit, the holding surface is blocked by an inclined section formed by the cut part of the center unit so that the inward or outward deviation of the additional unit is prevented.

Still another objective of the present disclosure is that the fastening part has an extension surface so that a portion of the center unit removed by the cut part can be restored by the extension surface.

Still another objective of the present disclosure is that the fastening part is formed in a non-porous structure so that the coupling strength of a coupling portion is increased and the overall rigidity of the augment is reinforced.

Still another objective of the present disclosure is that the additional unit has a fixing part, and through the coupling of the fixing part of the additional unit to the locking part of the center unit, the additional unit is stably fixed to the center unit.

Still another objective of the present disclosure is that a second recessed part recessed in the separating direction of the additional unit is formed to accommodate the first vertical part of the center unit in the second recessed part so that the additional unit can be separably fixed to the center unit.

Still another objective of the present disclosure is that the shape of the second recessed part has a shape complementary to the shape of the first vertical part so that the additional unit coupled to the center unit can stably maintain the coupled state.

Still another objective of the present disclosure is that a third vertical part protruding in the coupling direction of the additional unit is provided to generate additional fixing force due to the accommodation of the third vertical part in the first recessed part of the center unit so that fixing force between the center unit and the additional unit is further increased.

Still another objective of the present disclosure is that the vertical length of the third vertical part is shorter than the vertical depth of a space defined by the first recessed part to have an empty free space under the third vertical part, thereby preventing the problem that main coupling between the first vertical part and the second recessed part is not achieved when coupling between the third vertical part and the first recessed part is difficult due to tight tolerance, and the third vertical part can be more easily inserted into the first recessed part, thereby promoting the fast and accurate positioning of the center unit and the additional unit to each other.

Still another objective of the present disclosure is that the fixing part is formed in a non-porous structure so that the coupling strength of a coupling portion is increased and the overall rigidity of the augment is reinforced.

### Technical Solution

In order to accomplish the above objectives, the present disclosure provides a modular augment having the following configuration.

According to one embodiment of the present disclosure, the modular augment includes: a center unit having an accommodation space defined therein and having a first surface and a second surface which are open; an additional unit coupled to the center unit, wherein according to a degree of a patient's bone loss, the additional unit is selectively coupled to the center unit so as to adjust a degree of bone reinforcement.

According to another embodiment of the present disclosure, the center unit may include a coupling part configured to separably couple the additional unit to the center unit.

According to still another embodiment of the present disclosure, the coupling part may have a shape to allow the additional unit to be coupled to the center unit by sliding the additional unit in a first direction, and to allow the additional unit coupled to the center unit to be separated therefrom by sliding the additional unit in a second direction opposite to the first direction.

According to still another embodiment of the present disclosure, the coupling part may include a cut part formed by removing a side surface of the center unit toward the open second surface from the open first surface of the center unit so as to define space into which the additional unit is slidably inserted.

According to still another embodiment of the present disclosure, the coupling part may include a locking part formed to fix the additional unit inserted through the cut part.

According to still another embodiment of the present disclosure, the locking part may include: a first vertical part protruding in the separating direction of the additional unit so as to be accommodated in the additional unit; a first recessed part recessed in the coupling direction of the additional unit so as to accommodate the additional unit; and a second vertical part protruding in the separating direction of the additional unit so as to support the additional unit accommodated in the first recessed part.

According to still another embodiment of the present disclosure, the coupling part may be formed on each of medial and lateral sides of the center unit relative to a center vertical axis of the center unit.

According to still another embodiment of the present disclosure, the coupling part may be formed on a posterior side of the center unit relative to a center horizontal axis of the center unit.

According to still another embodiment of the present disclosure, the coupling part may be formed in a non-porous structure.

According to still another embodiment of the present disclosure, the center unit may include: a bone contact part which is connected to the coupling part and is in contact with a bone; and a bone non-contact part which is not in contact with the bone, wherein the bone contact part may be formed in a porous structure, and the bone non-contact part may be formed in a non-porous structure.

According to still another embodiment of the present disclosure, the additional unit may include an inclined part tapered in a coupling direction in which the additional unit is coupled to the center unit.

According to still another embodiment of the present disclosure, the inclined part may include: a first inclined part tapered at a first angle relative to a vertical axis of the additional unit; and a second inclined part tapered by extending from an end of the first inclined part at a second angle relative to the vertical axis, wherein the first angle may be smaller than the second angle.

According to still another embodiment of the present disclosure, the inclined part may be formed in a porous structure.

According to still another embodiment of the present disclosure, the additional unit may include a fastening part coupled separably to the coupling part.

According to still another embodiment of the present disclosure, the fastening part may include a holding surface extending to be inclined along an inclined section formed by a cut part of the center unit so that the additional unit is held by the inclined section so as not to deviate in medial and lateral directions of the center unit.

According to still another embodiment of the present disclosure, the fastening part may include an extension surface extending from an end of the holding surface along a profile of an inner circumferential surface of the center unit.

According to still another embodiment of the present disclosure, the fastening part may be formed in a non-porous structure.

According to still another embodiment of the present disclosure, the additional unit may include a fixing part coupled to the locking part.

According to still another embodiment of the present disclosure, the fixing part may include a second recessed part recessed in the separating direction of the additional unit so as to accommodate the first vertical part of the center unit.

According to still another embodiment of the present disclosure, the second recessed part may be formed in a shape complementary to a shape of the first vertical part.

According to still another embodiment of the present disclosure, the fixing part may include a third vertical part protruding in the coupling direction of the additional unit so as to be accommodated in the first recessed part of the center unit.

According to still another embodiment of the present disclosure, the third vertical part may be configured such that a vertical length of the third vertical part is shorter than a vertical depth of space defined by the first recessed part.

According to still another embodiment of the present disclosure, the fixing part may be formed in a non-porous structure.

### Advantageous Effects

The present disclosure can have the following effects by the above embodiments, components to described below, combination thereof, and use relationship thereof.

According to the present disclosure, the additional unit separably coupled to the center unit is provided, thereby providing a modular type augment capable of easily adjusting the degree of bone reinforcement by selectively coupling the additional unit to the center unit according to the degree of a patient's bone loss.

According to the present disclosure, when a patient's bone loss is large only on a medial side, a modular augment having an additional unit coupled to the medial side of the center unit is provided, when a patient's bone loss is concentrated on a lateral side, a modular augment having an additional unit coupled to the lateral side of the center unit is provided, and when bone loss is large on both the medial and lateral sides, a modular augment having additional units coupled to the medial and lateral sides of the center unit is provided.

According to the present disclosure, the additional units coupled to the medial and lateral sides of the center unit can be configured to have different sizes, thereby providing a customized augment to a patient having the different degrees of bone losses on the medial and lateral sides.

According to the present disclosure, a surgeon can transform the augment according to a patient's condition, thereby enabling easy revision surgery.

According to the present disclosure, a manufacturer can provide an augment that can cover various types of patients even though the types of units are minimized, thereby enabling manufacturing costs to be reduced, the number of products to be prepared for surgery by a user such as a hospital can be drastically reduced, thereby preventing the unnecessary costs and facilitating inventory management, and a patient can receive an optimal augment that is appropriate for the patient's condition.

According to the present disclosure, the additional unit is slid in the first direction so that the additional unit is coupled to the center unit, and the separation of the additional unit coupled to the center unit is performed only by sliding the additional unit in the second direction opposite to the first direction, thereby preventing the unintentional separation of the additional unit from an implanted augment due to the implant located in the second direction of an augment although the additional unit is separably coupled to the center unit.

According to the present disclosure, the center unit has the cut part formed on a side surface thereof, thereby enabling the additional unit to be separably inserted into the center unit through the cut part.

According to the present disclosure, the locking part is formed on the coupling directional end part of the additional unit, thereby allowing the additional unit to be finally fastened and fixed to the locking part after the additional unit inserted through the cut part moves in the coupling direction.

According to the present disclosure, the locking part includes the first vertical part protruding in the separating direction of the additional unit so that the locking part is accommodated in the additional unit, the first recessed part recessed in the coupling direction of the additional unit to accommodate the additional unit therein, and the second vertical part protruding in the separating direction of the additional unit to support the additional unit accommodated in the first recessed part, thereby allowing the center unit and the additional unit to be separably dovetail-coupled to each other.

According to the present disclosure, the coupling part is formed on each of the medial and lateral sides of the center unit so that the coupling part is offset to a posterior side by a predetermined angle, thereby allowing the augment having the additional unit coupled to the center unit to have a shape corresponding to the anatomical shape of a cancellous bone.

According to the present disclosure, the coupling part is formed in a non-porous structure, thereby increasing the coupling strength of a coupling portion and reinforcing the overall rigidity of the augment.

According to the present disclosure, the center unit is divided into a bone contact part in contact with a bone and a bone non-contact part which is not in contact with the bone so that the bone contact part is formed in a porous structure and the bone non-contact part is formed in a non-porous structure, and bone cement is inserted into the augment, thereby inducing strong coupling between the augment and the implant, and fusing the implanted augment and the bone to each other due to the promotion of spontaneous bone growth therebetween.

According to the present disclosure, the additional unit has an inclined part tapered in the coupling direction of the additional unit, thereby locating an implanted augment in a cortical bone.

According to the present disclosure, the first inclined part of the additional unit tapered at the first angle relative to the vertical axis of the additional unit, and the second inclined part tapered by extending from the end of the first inclined part at the second angle relative to the vertical axis of the additional unit are provided, and the first angle is smaller than the second angle, thereby allowing the anatomical shape of a bone having a part whose cross section rapidly decreases when the part moves away from a joint surface to be reflected in the augment.

According to the present disclosure, the inclined part of the additional unit is formed to have a porous structure, thereby realizing the coupling of a bone and the augment through the promotion of natural bone growth in a portion of the additional unit in contact with the bone.

According to the present disclosure, the additional unit has the fastening part, thereby allowing the fastening part to be separably coupled to the coupling part of the center unit.

According to the present disclosure, the fastening part has the holding surface and when the additional unit is coupled to the center unit, the holding surface is blocked by the inclined section formed by the cut part of the center unit, thereby preventing the inward or outward deviation of the additional unit.

According to the present disclosure, the fastening part has the extension surface, thereby restoring a portion of the center unit removed by the cut part by the extension surface.

According to the present disclosure, the fastening part is formed in a non-porous structure, thereby increasing the coupling strength of a coupling portion and reinforcing the overall rigidity of the augment.

According to the present disclosure, the additional unit has a fixing part, thereby allowing the additional unit to be stably fixed to the center unit through the coupling of the fixing part of the additional unit to the locking part of the center unit.

According to the present disclosure, a second recessed part recessed in the separating direction of the additional unit is formed to accommodate the first vertical part of the center unit in the second recessed part, thereby allowing the additional unit to be separably fixed to the center unit.

According to the present disclosure, the shape of the second recessed part has a shape complementary to the shape of the first vertical part, thereby allowing the additional unit coupled to the center unit to stably maintain the coupled state.

According to the present disclosure, a third vertical part protruding in the coupling direction of the additional unit is provided to generate additional fixing force due to the accommodation of the third vertical part in the first recessed part of the center unit, thereby further increasing fixing force between the center unit and the additional unit.

According to the present disclosure, the vertical length of the third vertical part is shorter than the vertical depth of a space defined by the first recessed part to have an empty free space under the third vertical part, thereby preventing the problem that main coupling between the first vertical part and the second recessed part is not achieved when coupling between the third vertical part and the first recessed part is difficult due to tight tolerance, and the third vertical part can be more easily inserted into the first recessed part, thereby promoting the fast and accurate positioning of the center unit and the additional unit to each other.

According to the present disclosure, the fixing part is formed in a non-porous structure, thereby increasing the coupling strength of a coupling portion and reinforcing the overall rigidity of the augment.

### Description of Drawings

FIG. 1 is a view illustrating a conventional tibial augment.
FIG. 2 is a perspective view of a modular augment according to one embodiment of the present disclosure.
FIG. 3 is an exploded perspective view of FIG. 2.
FIG. 4 is a cross-sectional view illustrating a center unit of FIG. 3.
FIG. 5 is a sectional view taken along line A-A' of FIG. 3.
FIG. 6 is a cross-sectional view illustrating an additional unit of FIG. 3.
FIG. 7 is a sectional view taken along line B-B' of FIG. 3.
FIG. 8 is a view illustrating the design process of the center unit and the additional unit.
FIG. 9 is a top plan view illustrating the coupled state of the center unit and the additional unit manufactured according to FIG. 8.
FIG. 10 is a view illustrating the design process of the center unit and the additional unit.
FIG. 11 is a sectional view illustrating the coupled state of the center unit and the additional unit manufactured according to FIG. 10.
FIG. 12 is a perspective view of a modular augment according to another embodiment of the present disclosure.
FIG. 13 is a view illustrating a state in which the modular augment is used according to the one embodiment of the present disclosure.
FIG. 14 is a view illustrating a state in which the modular augment is used according to the another embodiment of the present disclosure.

### Best Mode

Hereinbelow, exemplary embodiments of a modular augment according to the present disclosure will be described in detail with reference to the accompanying drawings. In the following description of the present disclosure, when it is determined that a detailed description of a known function or configuration may unnecessarily obscure the gist of the present disclosure, detailed description thereof will be omitted. Unless there is a special definition, all terms in this specification are the same as the general meaning of terms understood by those skilled in the art to which the present disclosure belongs, and when conflicting with the meaning of terms used in this specification, the terms used generally in the art follow the definition of the terms used herein.

In the modular augment 1 of the present disclosure, units are selectively coupled to each other according to the degree of the bone loss of a patient so that the degree of bone reinforcement can be adjusted. For example, when a patient's bone loss is large only on a medial side, a modular augment 1 having an additional unit 30 coupled to the medial side of a center unit 10 to be described later may be provided, when a patient's bone loss is concentrated on a lateral side of the center unit, a modular augment 1 having an additional unit 30 coupled to the lateral side of the center unit 10 may be provided, and when a patient's bone loss is large on both the medial and lateral sides, a modular augment having additional units 30 coupled to the medial and lateral sides of the center unit 10 may be provided.

In addition, the additional units 30 coupled to the medial and lateral sides of the center unit 10 may be configured to have different sizes, so a customized augment may be provided to a patient having different degrees of bone losses on the medial and lateral sides.

Accordingly, according to the modular augment 1 of the present disclosure, a surgeon can transform the augment according to a patient's condition, thereby enabling easy revision surgery, a manufacturer can provide an augment that can cover various types of patients even though the types of units are minimized, thereby enabling manufacturing costs to be reduced, the number of products to be prepared for surgery by a user such as a hospital can be drastically reduced, thereby preventing unnecessary costs and facilitating inventory management, and a patient can receive an optimal augment that is appropriate for the patient's condition.

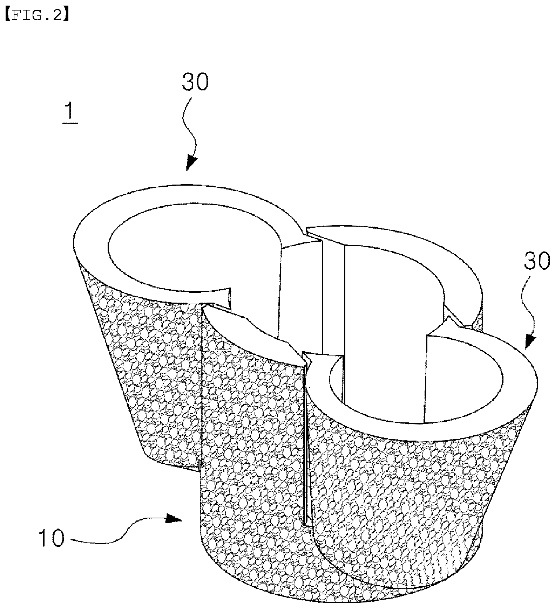

FIG. 2 is a perspective view of the modular augment 1 according to the embodiment of the present disclosure. Referring to FIG. 2, the modular augment 1 of the present disclosure includes the center unit 10 and the additional units 30.

The center unit 10 has an accommodation space 101 defined therein and is configured to be open in first and second surfaces thereof. As illustrated in FIG. 2, the center unit 10 may have the shape of a cylindrical pipe but may have a shape in which a predetermined portion of a side surface of the center unit 10 is removed.

A stem of a tibial implant or a femoral implant may be inserted into the accommodation space 101 of the center unit 10. It is not excluded that the size of the accommodation space 101 has a shape complementary to the size of the stem. However, when the stem of the implant is formed, an adapter may be added and offset the axis of the stem to one side. In view of this, the size of the accommodation space 101 may preferably be larger than the size of the stem.

The accommodation space 101 of the center unit 10 is filled with the bone cement so that the implant and the augment 1 can be coupled to each other. In addition, the accommodation space 101 of the center unit 10 may communicate with a seating space 301 formed inside of the additional unit 30 to be described later. In the case of the tibial implant, a keel is formed to connect the stem and a base plate, and the communication allows the keel to be inserted into the augment 1 without interference when the tibial implant and the augment 1 are coupled to each other.

It is preferable that the center unit 10 may be configured to be separably dovetail-coupled to the additional unit 30 to be described later.

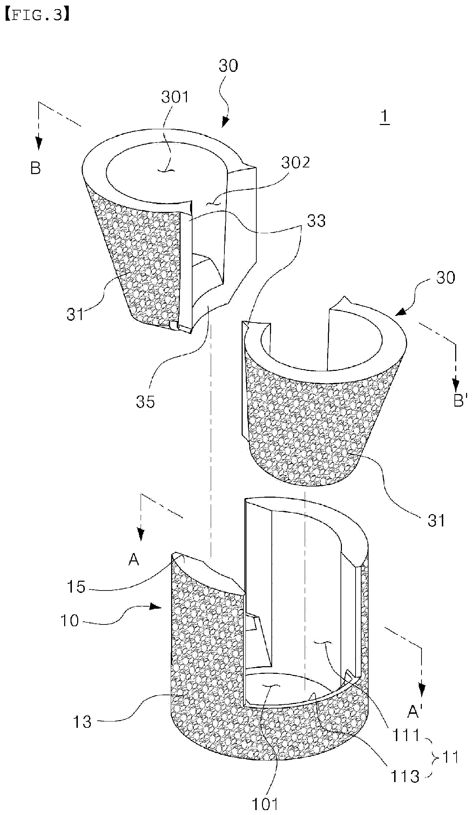

FIG. 3 is an exploded perspective view of FIG. 2. Referring to FIG. 3, the center unit 10 includes a coupling part 11, a bone contact part 13, and a bone non-contact part 15.

The coupling part 11 refers to a component for separably coupling the additional unit 30 to be described later to the center unit. The coupling part 11 may have a shape to allow the additional unit 30 to be coupled to the center unit 10 by sliding the additional unit 30 to be described later in a first direction, and to allow the additional unit 30 coupled to the center unit 10 to be separated therefrom by sliding the additional unit 30 in a second direction opposite to the first direction. Accordingly, the additional unit 30 is separably coupled to the center unit 10, and the implant is located in the second direction of the augment 1, and thus it is possible to prevent the unintentional separation of the additional unit 30 from the implanted augment 1.

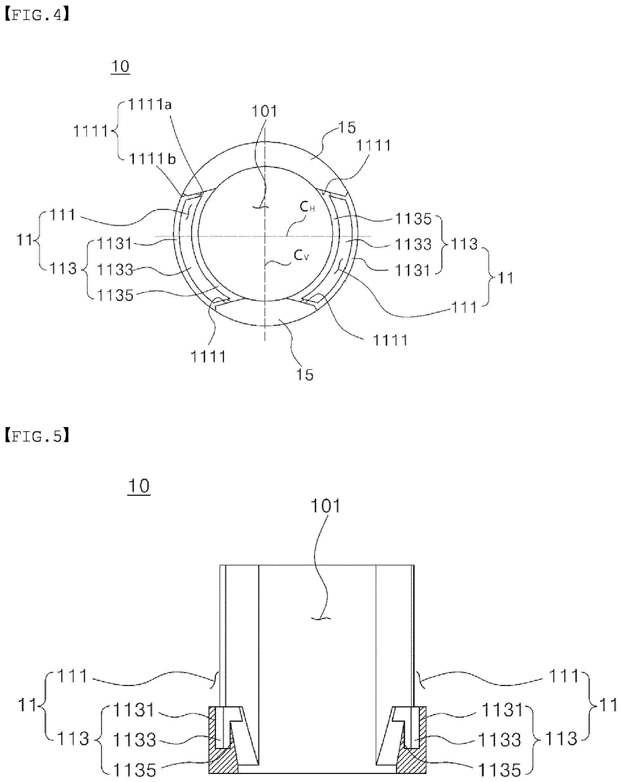

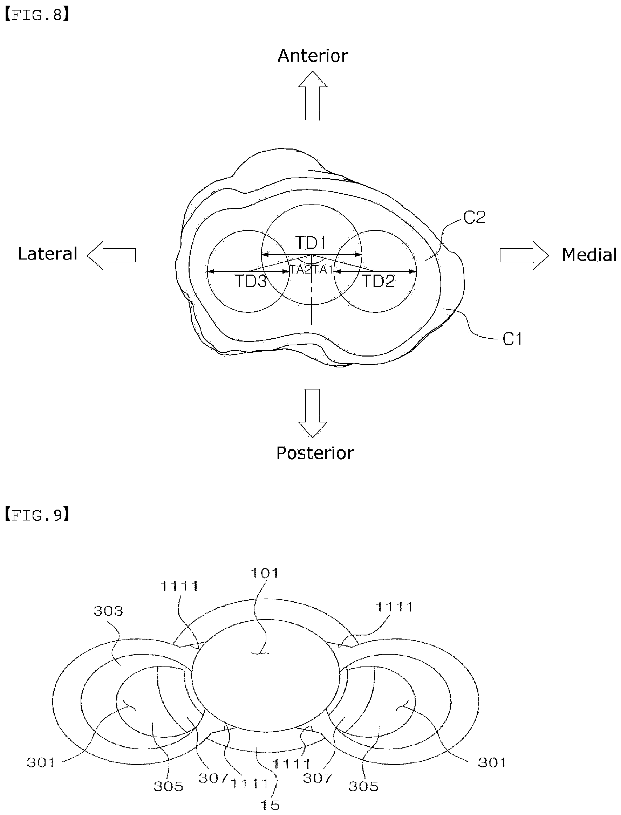

Referring to FIG. 4, the coupling part 11 is preferably formed on each of the medial and lateral sides of the center unit 10 relative to a center vertical axis C_{V} of the center unit 10 and on a posterior side of the center unit 10 relative to a center horizontal axis C_{H} of the center unit 10. Accordingly, the augment 1 in which the additional unit 30 is coupled to the center unit 10 may have a shape corresponding to the anatomical shape of the cancerous bone C2 as illustrated in FIG. 8.

When the coupling part 11 is formed in a porous structure having multiple pores, the coupling part 11 is relatively weak in rigidity and is likely to be damaged during the coupling process, such as broken or bent, so the coupling part 11 is preferably formed in a relatively strong non-porous structure.

The coupling part 11 includes a cut part 111 and a locking part 113.

The cut part 111 refers to a part in which a side surface of the center unit 10 is removed in a direction toward the open second surface from the open first surface of the center unit 10 so as to form space into which the additional unit 30 is slidably inserted.

The shape of the cut part 111 is not limited to any specific shape, but may have an approximate "U" shape as illustrated in FIGS. 3 and 4. As the cut part 111 is formed, the center unit 10 has the inclined section 1111 formed therein by the cut part 111 as illustrated in FIG. 4.

The inclined section 1111 may be divided into a first contact part 1111a and a second contact part 1111b. The first contact part 1111a is a part in contact with a holding surface 331 of a fastening part 33 to be described later when the additional unit 30 is coupled to the center unit 10, and prevents the additional unit 30 from deviating to the lateral side relative to the center of the center unit 10, and the second contact part 1111b prevents the additional unit 30 from deviating to the medial side in such a manner that the side surface of the additional unit 30 having a cone shape is supported by the second contact part 1111b despite medial external force toward the center of the center unit 10 when the additional unit 30 is coupled to the center unit 10.

In the center unit 10, the additional unit 30 is formed on each of the medial and lateral sides of the center unit 10 relative to the center vertical axis C_{V} of the center unit 10, and on the rather posterior side of the center unit 10 relative to the center horizontal axis C_{H} of the center unit 10. Accordingly, the cut part 111 may also formed on each of the medial and lateral sides by being biased to the posterior side as illustrated in FIG. 4.

The cut part 111 is fastened to the fastening part 33 of the additional unit 30 to be described later and guides the sliding of the additional unit 30 in the first direction or the second direction.

The cut part 111 is a part which meets the additional unit 30, and is preferably formed in a non-porous structure stronger than a porous structure so as to have a predetermined rigidity.

The locking part 113 refers to a component formed to fix the additional unit 30 inserted through the cut part 111. The additional unit 30 inserted under the guidance of the cut part 111 moves along a direction coupled to the center unit 10 and is finally fastened and fixed to the locking part 113. The locking part 113 may be separably coupled to a fixing part 35 of the additional unit 30 to be described later.

The locking part 113 is coupled to the fixing part 35 of the additional unit 30, and when the locking part is formed in a porous structure having a relatively low rigidity, the locking part 113 may be easily broken by external force, porous particles which are particles fallen off from the augment 1 are generated and cause various inflammatory reactions in a patient's body. Accordingly, the locking part 113 is preferably formed in a non-porous structure having a relatively high rigidity.

FIG. 5 is a sectional view taken along line A-A' of FIG. 3. Referring to FIG. 5, the locking part 113 includes a first vertical part 1131, a first recessed part 1133, and a second vertical part 1135.

As illustrated in FIG. 5, the first vertical part 1131 refers to a component protruding vertically in the separating direction of the additional unit 30 to be accommodated in a second recessed part 351 of the additional unit 30. When a third vertical part 353 of the additional unit 30 is inserted into the first recessed part 1133 to be described later, the first vertical part 1131 supports the inserted third vertical part 353 from the outside thereof. When the additional unit 30 is not lifted in the protruding direction of the first vertical part 1131, the additional unit 30 is not separated from the center unit 10.

As illustrated in FIG. 5, the first recessed part 1133 refers to a component recessed in the coupling direction of the additional unit 30 to accommodate the third vertical part 353 of the additional unit 30 therein. The shape of recessed space defined by the first recessed part 1133 may preferably have a shape complementary to the outer peripheral shape of the third vertical part 353 of the additional unit 30. Based on the first recessed part 1133, the first vertical part 1131 is formed on the outside, and the second vertical part 1135 to be described later is formed on the inside. The third vertical part 353 of the additional unit 30 seated in the first recessed part 1133 is supported in outer and inner surfaces by the first vertical part 1131 and the second vertical part 1135, respectively, and when the additional unit 30 is not lifted in the separating direction of the additional unit 30, the additional unit 30 is stably fixed to the center unit 10.

The second vertical part 1135 refers to a component protruding in the separating direction of the additional unit 30 to support the third vertical part 353 of the additional unit 30 accommodated in the first recessed part 1133. The outer peripheral surface of the second vertical part 1135 in contact with the third vertical part 353 of the additional unit 30 may have a shape complementary to the shape of the third vertical part 353. The shape of the inner peripheral surface of the second vertical part 1135 is not limited to any specific shape, but is inclined as illustrated in FIG. 5, and thus when a tibial implant is inserted into space in the augment 1, the augment 1 can be prevented from interfering with the keel of the tibial implant.

The bone contact part 13 is connected to the coupling part 11 of the center unit 10, and refers to a part in direct contact with a bone when the augment 1 connected to the implant is inserted into a patient's body. Referring to FIG. 3, the bone contact part 13 may preferably be formed in a porous structure. Since the bone contact part 13 is formed in a porous structure, the bone grows through micropores to strongly fuse the augment 1 and the bone to each other.

The bone non-contact part 15 refers to a part which is not in direct contact with a bone in a part connected to the coupling part 11 in the center unit 10 even though the augment 1 connected to the implant is implanted in a patient's body. The bone non-contact part 15 is formed in a non-porous structure. Since the bone non-contact part 15 is formed in a non-porous structure, the rigidity of the augment 1 can be reinforced through a solid non-porous structure so as to prevent the problem that the overall rigidity of the augment 1 decreases when the ratio of a porous structure increases.

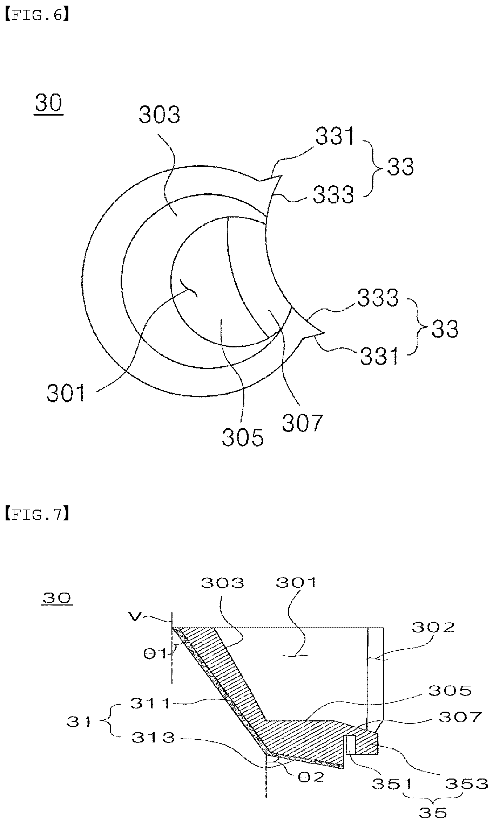

The additional unit 30 is a component coupled to the center unit 10, and the shape of the additional unit 30 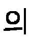 is not limited to any specific shape. However, as illustrated in FIG. 3, the additional unit 30 may be formed in the shape of a cone, specifically, in the shape of a truncated cone. FIG. 6 is a cross-sectional view illustrating the additional unit 30 of FIG. 3. As illustrated in FIG. 6, the additional unit 30 may have the seating space 301 formed therein, and have a communicating part 302 which is a part cut vertically on a side on which the additional unit 30 is coupled to the center unit 10, and thus the seating space 301 may communicate with the accommodation space 101 of the center unit 10 through the communicating part 302. Through the communication of the seating space 301 with the accommodation space 101, the stem of the tibial implant and a keel coupled to the base plate are seated in inner space defined by the augment 1 without interference.

Referring to FIG. 7, the additional unit 30 may include a first inclined surface 303 formed therein, a horizontal surface 305 extending horizontally from the end part of the first inclined surface 303, and a second inclined surface 307 extending slantingly from the end part of the horizontal surface 305. The first inclined surface 303, the horizontal surface 305, and the second inclined surface 307 are intended to prevent the interference of the augment 1 with the implant when the implant is accommodated in the augment 1, and may be transformed according to the shape of the implant accommodated in the seating space 301. The keel of the tibial implant is accommodated in the seating space 301 of FIG. 7, and the inner surface of the additional unit 30 is formed in a shape approximately similar to the shape of the accommodated keel.

Referring to FIG. 6 and 7, the additional unit 30 includes an inclined part 31, the fastening part 33, and the fixing part 35.

The inclined part 31 refers to a component tapered in a coupling direction in which the additional unit 30 is coupled to the center unit 10. When inserting the augment 1 into a patient's bone after forming the inclined part 31 having a tapered shape in the augment 1, a strong coupling force is generated, and the augment 1 has a shape corresponding to the anatomical shape of the bone. Accordingly, although the augment 1 is inserted into the patient's bone, the augment 1 can be stably positioned within a cortical bone without being exposed to the outside of the bone through the cortical bone. It is preferable that the inclined part 31 is formed in a porous structure. Through this, the bone and the augment 1 are coupled to each other through the promotion of natural bone growth in a part of the additional unit 30 in contact with the bone.

The inclined part 31 includes a first inclined part 311 and a second inclined part 313.

As illustrated in FIG. 7, the first inclined part 311 refers to an inclined surface of the additional unit 30 tapered at a first angle θ1 relative to the vertical axis V of the additional unit 30, and the second inclined part 313 refers to an inclined surface tapered by extending from the end of the first inclined part 311 at a second angle θ2 relative to the vertical axis V of the additional unit 30. Preferably, the first angle Θ 1 may be smaller than the second angle θ2. Anatomically, a bone has a part having a cross section rapidly decreasing when moving away from a joint surface, and the first inclined part 311 placed on a side close to the joint surface has a relatively small inclined angle, and the second inclined part 313, which has a larger inclined angle, is formed in a part in which a cross section rapidly decreases, so it is possible to provide an optimal augment 1 matching the anatomical shape of the bone. Accordingly, a boundary between the first inclined part 311 and the second inclined part 313, and the first angle θ1 and the second angle θ2 may be determined according to a patient's bone shape.

The fastening part 33 refers to a component coupled separably to the coupling part 11. As the cut part 111 having an approximate "U" shape is formed in the center unit 10, the inclined section 1111 including the first contact part 1111a and the second contact part 1111b is formed as illustrated in FIG. 4. The fastening part 33 has a shape as illustrated in FIG. 6 so that the fastening part 33 can be slidably fastened to the inclined section 1111. As illustrated in FIG. 3, the direction of the sliding may preferably be the direction of the longitudinal axis of the center unit 10. When the additional unit 30 is slidably coupled to the center unit 10, due to the unusual shape of the fastening part 33, the fastening part 33 can be stably fixed despite an external force applied to the center unit 10 or an external force applied in a direction opposite thereto.

The fastening part 33 is preferably formed in a non-porous structure. Since the fastening part 33 is formed in a non-porous structure, the coupling strength of a coupling portion may be increased and the overall rigidity of the augment 1 may be reinforced.

The fastening part 33 includes the holding surface 331 and the extension surface 333.

The holding surface 331 refers to a component extending to be inclined along the inclined section 1111 formed by the cut part 111 of the center unit 10 so that the additional unit 30 is held by the inclined section 1111 so as not to deviate in the medial and lateral directions of the center unit 10. When the additional unit 30 is coupled to the center unit 10, the holding surface 331 is blocked by the inclined section 1111 formed by the cut part 111 of the center unit 10, and accordingly, the holding surface 331 prevents the medial or lateral deviation of the additional unit 30. Specifically, in the inclined section 1111 including the first contact part 1111a and the second contact part 1111b, the first contact part 1111a is in contact with the holding surface 331 and prevents the additional unit 30 from deviating to the lateral side despite external force applied to the lateral side from the center of the center unit 10, and the second contact part 1111b prevents the additional unit 30 from deviating to the medial side in such a manner that the side surface of the additional unit 30 having a cone shape is supported by the second contact part 1111b despite a medial external force toward the center of the center unit 10.

The extension surface 333 is a component extending from the end of the holding surface 331 along the profile of the inner circumferential surface of the center unit 10 so that a portion of the center unit 10 removed by the cut part 111 can be restored by the extension surface 333. When the cut part 111 is formed in the center unit 10 to fasten the additional unit 30 thereto, a stem seated in the accommodation space 101 may not be supported appropriately, and particularly, when a surface surrounding the periphery of the accommodation space 101 is insufficient, a means preventing the leakage of bone cement filled in the accommodation space 101 to the outside is insufficient until the bone cement is cured, and accordingly, the formation of the extension surface 333 on the fastening part 33 compensates for such a problem.

The fixing part 35, which is configured to be coupled to the locking part 113, may be preferably formed in a non-porous structure. Since the fixing part 35 is formed in a non-porous structure, the coupling strength of a coupling portion can be increased, and the overall rigidity of the augment can be reinforced. Through coupling between the cut part 111 of the center unit 10 and the fastening part 33 of the additional unit 30, fixing force for fixing the additional unit 30 to the center unit 10 is first generated, and through coupling between the fixing part 35 of the additional unit 30 and the locking part 113 of the center unit 10, a second fixing force between the center unit 10 and the additional unit 30 is generated. Accordingly, although the center unit 10 and the additional unit 30 are formed to be separable from each other, more stable coupling can be maintained during the coupling of the center unit 10 and the additional unit 30.

The fixing part 35 includes the second recessed part 351 and the third vertical part 353.

The second recessed part 351 refers to a component recessed in the separating direction of the additional unit 30 to accommodate the first vertical part 1131 of the center unit 10. The first vertical part 1131 of the center unit 10 is accommodated in the second recessed part 351 so that the additional unit 30 can be separably coupled to the center unit 10. Preferably, the second recessed part 351 is formed in a shape complementary to the shape of the first vertical part 1131, through which the additional unit 30 coupled to the center unit 10 is more stably fixed.

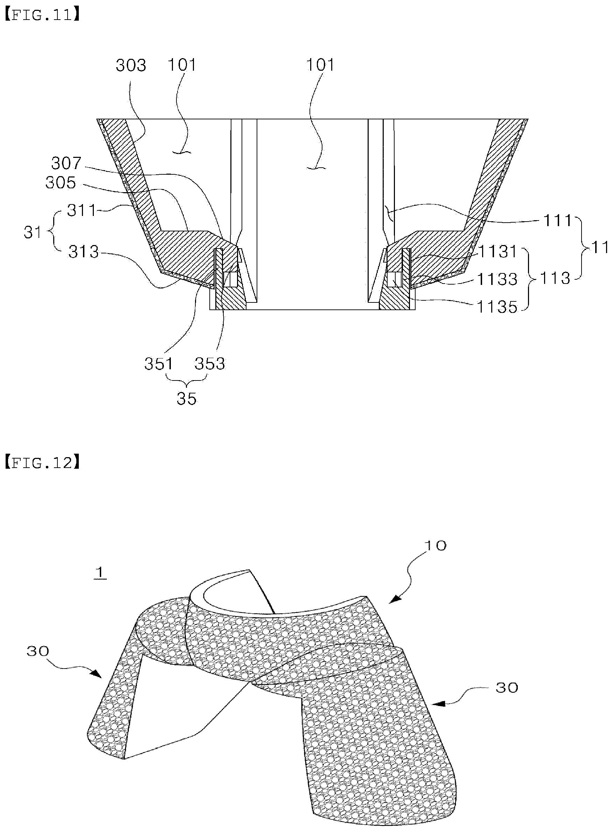

The third vertical part 353 refers to a component protruding in the coupling direction of the additional unit 30 so as to be accommodated in the first recessed part 1133 of the center unit 10. As the third vertical part 353 is accommodated in the first recessed part 1133 of the center unit 10 to generate additional fixing force, fixing force between the center unit 10 and the additional unit 30 can be further increased. The vertical length of the third vertical part 353 may be shorter than the vertical depth of space defined by the first recessed part 1133. Since the vertical length of the third vertical part 353 is shorter than the vertical depth of the space defined by the first recessed part 1133, there is empty free space under the third vertical part 353 as illustrated in FIG. 11, thereby the problem that main coupling between the first vertical part 1131 and the second recessed part 351 is not achieved when coupling between the third vertical part 353 and the first recessed part 1133 is difficult due to tight tolerance, and the third vertical part 353 can be more easily inserted into the first recessed part 1133, thereby promoting the fast and accurate positioning of the center unit 10 and the additional unit 30 to each other.

FIG. 8 is a view illustrating the design process of the center unit 10 and the additional unit 30. Referring to FIG. 8, the proximal end of the tibia T of the patient has an approximately oval shape as a whole, and a relatively strong cortical bone C1 with high bone density is formed on the outside of the bone, and a relatively weak cancellous bone C2 with low bone density is formed inside the bone. Since the combination of an implant and the augment is required to be inserted into the cancellous bone C2, the length of AP and the length of ML are preset so that the augment 1 can be located within the cancellous bone C2 area. Next, based on the center point of AP and ML, a diameter of TD1 is drawn, and in consideration of the angle of the keel of the tibial implant, a line having an angle TA1 and a line having an angle TA2 are drawn, and then a circle having a diameter of TD2 located on the medial side and a circle having a diameter of TD3 located on the lateral side are respectively modeled by setting center points on corresponding lines so that intersecting areas occur.

FIG. 9 is a top plan view illustrating the coupled state of the center unit 10 and the additional unit 30 manufactured according to FIG. 8. Referring to FIG. 9, the circle having the diameter TD1, the circle having the diameter TD2, and the circle having the diameter TD3, which are made according to the above-mentioned modeling process of FIG. 8, respectively correspond to the planar shapes of the center unit 10, the additional unit 30 coupled on the medial side, and the additional unit 30 coupled on the lateral side. Through the modeling process of FIG. 8, the position of the cut part 111 offset in a posterior direction from the center unit 10 may be determined, and the shape of the fastening part 33 of the additional unit 30 slidably fastened to the cut part 111 may be determined.

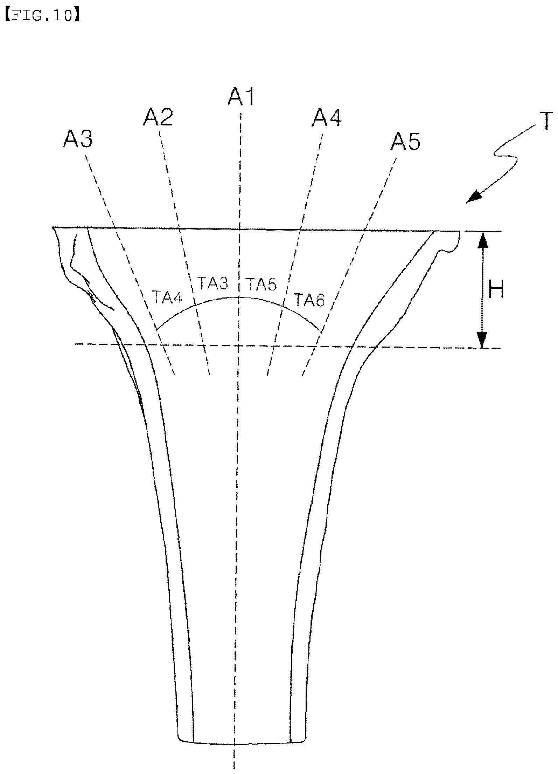

FIG. 10 illustrates the design process of the center unit 10 and the additional unit 30. Referring to FIG. 10, when a drilling axis A1 for inserting the center unit 10 is preset, a drilling axis A2 inclined at an angle TA3 from the axis A1 is preset, and the boundary A3 of the outer peripheral surface of the augment 1 inclined at an angle TA4 from the axis A2 is determined. In a similar way, on the opposite side, a drilling axis A4 inclined at an angle TA5 from the axis A1 is preset, and the boundary A5 of the outer peripheral surface of the augment 1 inclined at an angle TA6 from the axis A4 is determined. In addition, the height of the augment 1 is determined by determining depth H from the resected proximal end of the tibia.

FIG. 11 is a sectional view illustrating the coupled state of the center unit and the additional unit manufactured according to FIG. 10. Referring to FIG. 11, the central axis of the center unit 10 is preset through the A1 of FIG. 10, the central axis of the seating space of the additional unit 30 at a first side is preset through the A2, and the inclined part 31 of the additional unit 30 at the first is preset through the A3. In addition, the central axis of the seating space of the additional unit 30 at a second side may be present through the A4, and the inclined part 31 of the additional unit 30 at the second side may be preset through the A5.

FIG. 12 illustrates a perspective view of the modular augment 1 according to another embodiment of the present disclosure. FIG. 12 illustrates the modular augment 1 coupled to a femoral implant. Referring to FIG. 12, the modular augment 1 coupled to the femoral implant also has the additional units 30 suitable for a patient's bone state selectively and separably coupled to the opposite sides of the center unit 10. When required, the additional units 30 may be slidably coupled to the center unit 10, additional units 30 having different sizes may be respectively coupled to the additional units 30 at medial and lateral sides, and the modular augment 1 may have additional empty spaces to accommodate a protruding part formed on the joint contact surface of the femoral implant having a shape different from the shape of the tibial implant.

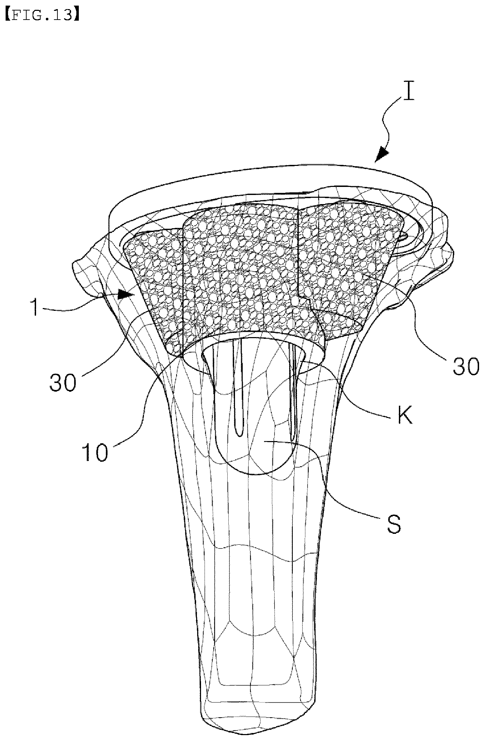

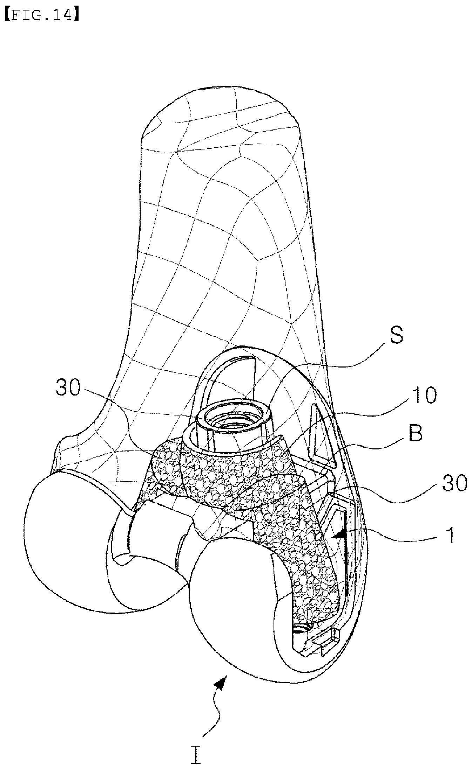

FIG. 13 illustrates a state in which the modular augment 1 coupled to the tibial implant I is inserted into a patient's tibia, and FIG. 14 illustrates a state in which the modular augment 1 coupled to the femoral implant I is inserted into a patient's femur.

First, referring to FIG. 13, in the tibial implant I, keels K are formed on the opposite sides of the stem S, and the modular augment 1 is assembled with the tibial implant I by having a shape which can accommodate these keels K. In addition, the inner space of the modular augment 1 is filled with the bone cement so that the modular augment 1 can be easily fixed to the tibial implant I. The tibial implant I coupled to the modular augment 1 is implanted inside a patient's bone, and a portion of the modular augment 1 in contact with the bone is formed to have a porous structure, and thus bone growth between the augment 1 and the bone is promoted so that the augment 1 and the bone are fused to each other.

Referring to FIG. 14, a stem S is formed even in the femoral implant I. The protruding stem S is accommodated in the center unit 10, and the additional units 30 are formed on the opposite sides of the center unit 10 to reinforce a patient's bone. When a patient's bone loss is concentrated only on a first side of the center unit 10, the additional unit 30 is coupled only to the first side, and the additional unit 30 may not be coupled to the second side of the center unit 10. As illustrated in FIG. 14, the femoral implant I may have a protruding box B formed near the joint contact surface of the femoral implant I, and thus in order to prevent interference, the modular augment 1 coupled to the femoral implant I may have an additional empty space to accommodate the protruding box B.

The above detailed description is to illustrate the present disclosure. In addition, the foregoing is intended to illustrate the exemplary embodiments of the present disclosure, and the present disclosure may be used in various combinations, variations, and environments. That is, changes or modifications are possible within the scope of the inventive concept disclosed in this specification, within a scope equivalent to the present disclosure and/or within the scope of skill or knowledge in the art. The embodiments of the present disclosure describes a best state for realizing the technical idea of the present disclosure, and various changes required in the specific application field and purpose of the present disclosure are also possible. Accordingly, the above detailed description of the present disclosure is not intended to limit the present disclosure to the disclosed embodiments. In addition, the appended claims should be construed to cover other embodiments as well.

## Claims

1. A modular augment comprising:
a center unit having an accommodation space defined therein and having a first surface and a second surface which are open;
an additional unit coupled to the center unit,
wherein according to a degree of a patient's bone loss, the additional unit is selectively coupled to the center unit so as to adjust a degree of bone reinforcement.

2. The modular augment of claim 1, wherein the center unit comprises a coupling part configured to separably couple the additional unit to the center unit.

3. The modular augment of claim 2, wherein the coupling part has a shape to allow the additional unit to be coupled to the center unit by sliding the additional unit in a first direction, and to allow the additional unit coupled to the center unit to be separated therefrom by sliding the additional unit in a second direction opposite to the first direction.

4. The modular augment of claim 3, wherein the coupling part comprises a cut part formed by removing a side surface of the center unit toward the open second surface from the open first surface of the center unit so as to define space into which the additional unit is slidably inserted.

5. The modular augment of claim 4, wherein the coupling part comprises a locking part formed to fix the additional unit inserted through the cut part.

6. The modular augment of claim 5, wherein the locking part comprises:
a first vertical part protruding in the separating direction of the additional unit so as to be accommodated in the additional unit;
a first recessed part recessed in the coupling direction of the additional unit so as to accommodate the additional unit; and
a second vertical part protruding in the separating direction of the additional unit so as to support the additional unit accommodated in the first recessed part.

7. The modular augment of claim 2, wherein the coupling part is formed on each of medial and lateral sides of the center unit relative to a center vertical axis of the center unit.

8. The modular augment of claim 7, wherein the coupling part is formed on a posterior side of the center unit relative to a center horizontal axis of the center unit.

9. The modular augment of claim 2, wherein the coupling part is formed in a non-porous structure.

10. The modular augment of claim 9, wherein the center unit comprises: a bone contact part which is connected to the coupling part and is in contact with a bone; and a bone non-contact part which is not in contact with the bone,
wherein the bone contact part is formed in a porous structure, and the bone non-contact part is formed in a non-porous structure.

11. The modular augment of claim 1, wherein the additional unit comprises an inclined part tapered in a coupling direction in which the additional unit is coupled to the center unit.

12. The modular augment of claim 11, wherein the inclined part comprises: a first inclined part tapered at a first angle relative to a vertical axis of the additional unit; and a second inclined part tapered by extending from an end of the first inclined part at a second angle relative to the vertical axis,
wherein the first angle is smaller than the second angle.

13. The modular augment of claim 11, wherein the inclined part is formed in a porous structure.

14. The modular augment of claim 2, wherein the additional unit comprises a fastening part coupled separably to the coupling part.

15. The modular augment of claim 14, wherein the fastening part comprises a holding surface extending to be inclined along an inclined section formed by a cut part of the center unit so that the additional unit is held by the inclined section so as not to deviate in medial and lateral directions of the center unit.

16. The modular augment of claim 15, wherein the fastening part comprises an extension surface extending from an end of the holding surface along a profile of an inner circumferential surface of the center unit.

17. The modular augment of claim 14, wherein the fastening part is formed in a non-porous structure.

18. The modular augment of claim 6, wherein the additional unit comprises a fixing part coupled to the locking part.

19. The modular augment of claim 18, wherein the fixing part comprises a second recessed part recessed in the separating direction of the additional unit so as to accommodate the first vertical part of the center unit.

20. The modular augment of claim 19, wherein the second recessed part is formed in a shape complementary to a shape of the first vertical part.

21. The modular augment of claim 18, wherein the fixing part comprises a third vertical part protruding in the coupling direction of the additional unit so as to be accommodated in the first recessed part of the center unit.

22. The modular augment of claim 21, wherein the third vertical part is configured such that a vertical length of the third vertical part is shorter than a vertical depth of space defined by the first recessed part.

23. The modular augment of claim 18, wherein the fixing part is formed in a non-porous structure.
